(19) Europäisches Patentamt
European Patent Office
Office européen des brevets

(11) **EP 3 824 926 B1**

(12) **EUROPEAN PATENT SPECIFICATION**

(45) Date of publication and mention
of the grant of the patent:
**31.01.2024   Bulletin 2024/05**

(21) Application number: **19210762.1**

(22) Date of filing: **21.11.2019**

(51) International Patent Classification (IPC):
***A61M 5/31*** *(2006.01)*

(52) Cooperative Patent Classification (CPC):
**A61M 5/3129;** A61M 2005/3131

(54) **RECEPTACLE FOR PHARMACEUTICAL PACKAGING HAVING A GRADUATED LUBRICANT LAYER**

BEHÄLTER FÜR PHARMAZEUTISCHE VERPACKUNG MIT EINER ABGESTUFTEN SCHMIERMITTELSCHICHT

RÉCIPIENT D'EMBALLAGE PHARMACEUTIQUE COMPORTANT UNE COUCHE DE LUBRIFIANT GRADUÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.05.2021   Bulletin 2021/21**

(73) Proprietor: **SCHOTT Pharma AG & Co. KGaA**
**55122 Mainz (DE)**

(72) Inventors:
• **Moser, Raymond**
**9032 Engelburg (CH)**

• **Mangold, Stephanie**
**55288 Schornsheim (DE)**

(74) Representative: **Herzog IP Patentanwalts GmbH**
**Steinstraße 16-18**
**40212 Düsseldorf (DE)**

(56) References cited:
**EP-A1- 2 216 061        WO-A1-2014/190225**
**WO-A1-2015/181173**

**Description**

Field of the Invention

The invention generally relates to a receptacle for pharmaceutical packaging having a graduated layer of lubricant. In particular, the invention relates to a receptacle; a kit comprising a receptacle and a charge; an assembly comprising a receptacle, a charge and a liquid pharmaceutical composition; a process for the preparation of a receptacle and a use of a lubricant layer of graduated thickness.

Background

Pharmaceutical material can be provided in a number of forms and contained in a variety of different containers. In the case of a liquid pharmaceutical material, some common examples are ampules, vials, cartridges and syringes. One widely used format employs a sliding plunger within a container for ejecting a liquid out of an aperture. One approach is to provide a lubricating layer on the inside of the container to facilitate sliding movement of the plunger.

U.S. Patent Application Document No. 4,767,414 A describes plasma activation of an inner surface prior to application of a layer of silicone lubricant.

European Patent No. EP 0920879 B1 describes a recipe for a silicone-based mixture comprising reactive components and unreactive components.

WO 2015/181173 A1 discloses a method for storing an emulsion-adjuvanted vaccine in a medical injection device comprising a lubricant coating, said method comprising providing a medical injection device comprising a barrel having an inner surface, forming a silicone oil layer on at least a part of the inner surface of the barrel that is intended to be in contact with the vaccine, carrying out an oxidizing plasma treatment of said layer so as to crosslink at least part of the silicone oil layer and form a lubricant coating, and filling the barrel with the emulsion-adjuvanted vaccine.

EP 2 216 061 A1 discloses a syringe comprising a syringe barrel which includes an inner surface provided with a structure in which a sliding resistance is changed step by step upon sliding a piston.

There persists a need for improved approaches to lubrication of pharmaceutical containers, in particular for delivering multiple separate doses from a single container.

Summary of the Invention

It is an object of the invention to at least partially solve one or more of the above-mentioned challenges.

It is an object of the invention to provide an improved receptacle for pharmaceutical packaging, in particular for multi-dose delivery.

It is an object of the invention to provide an improved kit comprising a receptacle and a charge, in particular for multi-dose delivery.

It is an object of the invention to provide an improved process for preparing a receptacle for pharmaceutical packaging, in particular for multi-dose delivery.

It is an object of the invention to provide an improved assembly comprising a receptacle, a charge and a liquid pharmaceutical composition, in particular for multi-dose delivery.

It is an object of the invention to provide a receptacle for pharmaceutical packaging having improved stiction properties, in particular for multi-dose delivery.

It is an object of the invention to provide a kit having improved stiction properties, in particular for multi-dose delivery.

It is an object of the invention to provide a process for preparing a receptacle for pharmaceutical packaging providing improved stiction properties, in particular for multi-dose delivery.

It is an object of the invention to provide an assembly having improved stiction properties, in particular for multi-dose delivery.

The invention is defined in independent claims 1 and 13. Advantageous embodiments are set out in dependent claims 2-12

The object of the invention is approached by a receptacle for pharmaceutical packaging, the receptacle having an elongate barrel section and comprising a first aperture at a first end and a second aperture at a second end, wherein:

a. the elongate barrel section has a direction of elongate extension and an axis in the direction of elongate extension;
b. an axial position $p$ is determined along the axis;
c. the elongate barrel section extends from an axial position $p_A$ to an axial position $p_B$, wherein the receptacle is adapted and arranged for ejecting a liquid pharmaceutical composition from the second end and wherein $p_B$ is closer to the second end than is $p_A$;

d. an elongate barrel section length $L_B$ is the distance between $p_A$ and $p_B$;

e. the receptacle has a side wall extending over the elongate barrel section, the side wall having an inner surface bordering an interior, the interior having a diameter;

f. a layer of a lubricant is located on at least a part of the inner surface;

g. at a given axial position p on the axis between $p_A$ and $p_B$, the following:

> i. the thickness of the side wall,
> ii. the thickness of the layer, and
> iii. the diameter of the interior,

are each determined as an angular mean in a cross-sectional plane perpendicular to the axis at the axial position p;

h. a portion X of the axis extends from an axial position $p_1$ to an axial position $p_2$ such that the following criteria are satisfied:

> i. both $p_1$ and $p_2$ lie between $p_A$ and $p_B$,
> ii. a portion length $L_X$ is the distance between $p_1$ and $p_2$,
> iii. $L_X$ is at least a quarter of $L_B$, preferably at least a half of $L_B$, more preferably at least 80% of $L_B$, most preferably at least 90% of $L_B$,
> iv. the layer extends over the entire portion X, and
> v. a thickness $t_1$ of the layer at $p_1$ is less than a thickness $t_2$ of the layer at $p_2$;

wherein $p_1$ is closer to $p_A$ than is $p_2$ and wherein the thickness of the layer increases monotonically over the portion from $p_1$ to $p_2$

i. criteria i., ii., iii. and iv.:

> i. The length $L_B$ is in the range from 3 to 20 cm, preferably in the range from 4 to 15 cm, more preferably in the range from 5 to 12 cm, most preferably 6 to 8 cm;
> ii. The mean value of the diameter of the interior determined over the range $p_A$ to $p_B$ is in the range from 0.4 to 4 cm, preferably in the range from 0.6 to 3 cm, more preferably in the range from 0.8 to 2.5 cm, most preferably 1 to 2 cm;
> iii. The mean thickness of the sidewall determined over the range $p_A$ to $p_B$ is in the range from 0.3 to 4 mm, preferably in the range from 0.7 to 3 mm, more preferably in the range from 1.1 to 2 mm, most preferably in the range from 1.4 to 1.8 mm; and
> iv. The volume of the interior is in the range from 0.1 to 150 ml, preferably in the range from 0.5 to 50 ml, more preferably in the range from 1 to 25 ml, most preferably 2 to 10 ml, most preferably 3 to 5 ml;

are satisfied.

According to a preferred embodiment of the receptacle the ratio of the thicknesses $t_1:t_2$ is in the range from 1:1.1 to 1:100, preferably in the range from 1:5 to 1:90, more preferably in the range from 1:10 to 1:80, most preferably in the range from 1:15 to 1:70.

According to a further preferred embodiment of the receptacle the lubricant comprises one or more silicone oils. Preferably, the lubricant comprises in total at least 5 wt. %, more preferably at least 15 wt. %, most preferably at least 25 wt. % of one or more silicone oils, based on the total weight of the lubricant of the layer.

According to a further preferred embodiment of the receptacle the one or more silicone oils are at least partially contained in a matrix, wherein the matrix is bound to the inner surface.

According to a further preferred embodiment of the receptacle the matrix is a polymer, preferably a crosslinked polymer. Preferably, the polymer comprises SiO-containing repeating units. A preferred polymer is a polysiloxane, more preferably a crosslinked polysiloxane.

According to a further preferred embodiment of the receptacle the interior is cylindrical or truncated conical over the elongate barrel section. A preferred truncated cone has a cone aperture in the range from 0.04° to 0.4°, preferably in the range from 0.08° to 0.25°, more preferably in the range from 0.1° to 0.2°.

According to a further preferred embodiment of the receptacle the first aperture has a greater area than the second aperture, preferably at least 50% greater, more preferably at least 100% greater, most preferably at least 200% greater, based on the surface area of the second aperture. In an alternative embodiment, the receptacle comprises a first aperture at a first end and a dead end at a second end.

According to a further preferred embodiment of the receptacle the internal diameter at $p_1$ is $d_1$ and the internal

diameter at $p_2$ is $d_2$ and $d_1$ is greater than $d_2$. $d_1$ is preferably at least 0.05% greater than $d_2$, more preferably at least 0.1%, most preferably at least 0.2%, based on the diameter $d_2$. $d_1$ is preferably up to 5% greater than $d_2$, preferably up to 4%, more preferably up to 3%, based on the diameter $d_2$.

According to a further preferred embodiment of the receptacle the receptable comprises an aperture and an attachment means at the aperture. Preferably, an attachment means is at an end, more preferably at a second end. A preferred attachment means is adapted and arranged for attaching one or both selected from the group consisting of a needle and a tube. Some preferred attachment means are a screw thread, a latch, a Luer fitting and a bayonet-style fitting.

According to a further preferred embodiment of thee receptacle the layer has a minimum thickness $t_{min}$ determined in the portion X of at least 60 nm, preferably at least 90 nm, more preferably at least 100 nm, most preferably at least 110 nm.

The object of the invention is also approached by a kit comprising as kit parts:

> a. the receptacle according to any of the above-mentioned embodiments, and
> b. a charge;

wherein the charge is adapted and arranged to be positioned in the interior such that:

> i. the charge seals a cross section of the interior between the inner surfaces of the side walls;
> ii. the charge has a front end at the axial position closest to $p_B$ at which the charge contacts the layer or the inner surface;
> iii. the charge has a back end at the axial position closest to $p_A$ at which the charge contacts the layer or the inner surface;
> iv. a length of the charge $L_C$ is the distance between the front end and the back end;
> v. the charge has a charge axial position, being the axial position of the front end;
> vi. the charge is movable in a direction parallel to the axis with a stiction s, s being a function of the charge axial position.

A preferred charge comprises an elastomeric material.

According to a preferred embodiment of the kit for charge axial positions in the range from $p_1 + L_C$ to $p_2$, the stiction s has a maximum value $s_{max}$, a minimum value $s_{min}$ and the value of $s_{min} / s_{max}$ is 40 % or more, more preferably 50 % or more, more preferably 60 % or more, more preferably 65 % or more, more preferably 70 % or more, more preferably 75 % or more, most preferably 80 % or more.

According to a further preferred embodiment the kit the stiction at charge axial position $p_2$ is equal to or less than the stiction at charge axial position $p_1 + L_C$. In one aspect of this embodiment, the stiction at charge axial position $p_2$ less than the stiction at charge axial position $p_1 + L_C$ by at least 5%, preferably at least 10%, more preferably at least 15%, most preferably at least 20%, based on the stiction at charge axial position $p_1 + L_C$.

The object of the invention is also approached by an assembly comprising a receptacle according to any of the above-mentioned embodiments and a charge, wherein:

> a. the charge is positioned in the interior sealing a cross section of the interior;
> b. the interior contains a liquid pharmaceutical composition, located between the sealed cross section and the aperture; and
> c. the assembly is adapted and arranged for the liquid pharmaceutical composition to be ejected through the aperture by movement of the charge in a direction parallel to the axis.

The object of the invention is also approached by a process for preparing a receptacle according to the invention, a kit according to the invention, or an assembly according to the invention, comprising a process step of applying the layer of lubricant by spreading with a spreading tool.

<u>Diameters, layer thicknesses and roughness</u>

The axis of the receptacle is used to determine axial position. At a given axial position, the side wall is a perimeter having a thickness lying in a cross-sectional plane perpendicular to the axis, likewise for the lubricant layer. Internal diameter, thickness of the lubricant layer, thickness of the side wall and surface roughness at a given point along the axis are preferably mean values determined around a perimeter. A mean around a perimeter is an angular mean. An angular mean is preferably determined by measuring at 8 points around the perimeter, the 8 points being separated by equal angles.

Monotonic parameters

Herein, the term "monotonic", when applied to a variable dependent on p, preferably ignores sharp variations in the function with small changes in p, otherwise known as roughness. Monotonicity for a parameter Y over the portion X is preferably determined as follows:

- The portion X is split into n sections of equal length: $M_1$ to $M_n$;
- $M_1$ to $M_n$ are ordered, with $M_1$ starting at $p_1$ and $M_n$ finishing at $p_2$;
- The mean values of Y over the sections $M_1$ to $M_n$ are $Y_1$ to $Y_n$ respectively;
- For a monotonic increase, each mean value of Y through the series $Y_2$ to $Y_n$ is greater than the preceding mean value of Y: $Y_1$ to $Y_{n-1}$ respectively;
- For a monotonic decrease, each mean value of Y through the series $Y_2$ to $Y_n$ is less than the preceding mean value of Y: $Y_1$ to $Y_{n-1}$ respectively;
- x is an integer of 2 or more. Preferred values for x are 5, 10, 15 and 20.

Receptacle

A preferred receptacle is adapted and arranged to contain a pharmaceutical liquid. Some preferred receptacles are syringes and syringe barrels.

The receptacle has a first aperture at a first end and a second aperture at a second end. Another type of preferred receptacle has a dead end at a second end.

Elongate Barrel Section

The receptacle has an elongate barrel section. The elongate barrel section denominates a section of the receptacle. The receptacle may have further sections outside of the elongate barrel section. Another term for an elongate barrel section is a tube section. A preferred elongate barrel section is tubular.

Possible embodiments of the elongate barrel section and the axis are described herein in mathematical terms, for example as axes of symmetry, rotation or revolution, surfaces and solids of revolution and shapes such as cylinders and truncated cones. These embodiments are to be understood as allowing some variation from these precise mathematical concepts. Suitable variations from the mathematic concepts are those which do not inhibit the receptacle from functioning as a plunger system in cooperation with a charge.

The elongate barrel section has an axis. The axis may be an axis of rotation for the elongate barrel section. The axis may be an axis of revolution for the elongate barrel section. The side wall may be a solid of revolution about the axis. The inner surface may be a surface of revolution about the axis. The layer may be a solid of revolution about the axis.

The axis defines an axial position p. The axial position p is an axial position along the axis. In this document, the symbol p denotes an axial position in general terms and specific axial positions are denoted by the letter p with a subscript.

An axial position p along the axis is used as a parameter to describe the locations of points or cross-sections along the elongate barrel section, for example on the side wall. The axial position of a point not on the axis is found by projecting the point onto the axis by a displacement vector perpendicular to the axis. A cross section is a plane perpendicular to the axis. The axial position of a cross section is found at the point where the cross section meets the axis.

The elongate barrel section extends from an axial position $p_A$ to an axial position $p_B$. The elongate barrel section is contained by a cross section at $p_A$ and a cross section at $p_B$.

The receptacle has a first end and a second end, the receptacle is adapted and arranged for ejecting a liquid pharmaceutical composition from the second end and $p_B$ is closer to the second end than is $p_A$.

The receptacle has a side wall extending over the elongate barrel section. The side wall has an inner surface. The inner surface borders an interior. Preferred shapes for the side wall are a hollow cylinder, a hollow prism and a hollow truncated cone. A preferred hollow truncated cone has a diameter which decreases from $p_A$ to $p_B$. Preferred shapes for the interior are a cylinder, a prism and a truncated cone. A preferred truncated cone has a diameter which decreases from $p_A$ to $p_B$.

The inner surface is preferably smooth, but may have some roughness.

The thickness of the side wall is preferably measured as a difference in radial distance from the axis of the inner surface and an outer surface of the side wall.

The thickness of the side wall is preferably measured as a difference in radial distance from the axis of an inner surface of the layer and the inner surface of the side wall.

Preferred materials for the side wall are polymers and glasses.

In one embodiment, the side wall comprises a polymer, preferably is made out of a polymer. The polymer is preferably one or both selected from the group consisting of: one or more cyclic olefin copolymers and one or more cyclic olefin polymers. In one aspect of this embodiment, the polymer is preferably at least 30 wt. % of the side wall, more preferably at least 50 wt. %, more preferably at least 80 wt. %, most preferably about 100 wt. %.

In one embodiment, the side wall comprises a glass, preferably is made of a glass. A preferred glass in this context comprises one or more selected from the group consisting of: silicon, boron and aluminium. One preferred glass comprises boron and silicon. One preferred glass is a borosilicate glass. One preferred glass comprises aluminium and silicon. One preferred glass is an aluminosilicate glass. In one aspect of this embodiment, the glass is preferably at least 30 wt. % of the side wall, more preferably at least 50 wt. %, more preferably at least 80 wt. %, most preferably about 100 wt. %.

Lubricant layer

The layer of lubricant is located on the inner surface of the side wall. The lubricant layer may extend over the entire elongate barrel section or just a part of it. The lubricant layer extends over the entire portion X.

A preferred lubricant is a silicone-based lubricant.

A preferred lubricant comprises one or more polysiloxanes.

A preferred lubricant comprises one or more silicone oils, preferably a total content of silicone oils in the range from 10 to 50 wt. %, more preferably in the range from 20 to 40 wt. %, most preferably in the range from 25 to 35 wt. %, based on the total weight of the lubricant. A preferred silicone oil is a poly dimethyl silicone.

A preferred lubricant comprises a crosslinked polysiloxane matrix, preferably a total content of crosslinked polysiloxane matrix in the range from 50 to 90 wt. %, more preferably in the range from 60 to 80 wt. %, most preferably in the range from 65 to 75 wt. %, based on the total weight of the lubricant.

A preferred lubricant may be prepared from a mixture comprising one or more, preferably all, of the following:

- a reactive polysiloxane
- an unreactive polysiloxane
- a catalyst
- a diluent

A preferred reactive polysiloxane is adapted and arranged to undergo a cross-linking reaction to obtain a cross-linked network. The cross-linking may be catalysed by the catalyst.

A preferred unreactive polysiloxane does not undergo a cross-linking reaction. A preferred unreactive polysiloxane comprises one or more alkyl groups. A further preferred unreactive polysiloxane is fully substituted with alkyl groups.

A preferred catalyst catalyses a reaction to cross-link polysiloxanes.

A preferred diluent solves one or more of the other constituents of the mixture. A preferred diluent is silicon based. A preferred diluent is a short chain polysiloxane, preferably having 6 repeat units or less. A preferred diluent is hexamethyl disiloxane.

A preferred lubricant contains not more than 10 wt. % water, based on the total weight of the lubricant, preferably not more than 5 wt. %, more preferably not more than 1 wt. %.

The lubricant layer has a thickness profile from $p_1$ to $p_2$. The thickness of the layer at $p_2$ is greater than the thickness of the layer at $p_2$, preferably by at least 100 nm, more preferably by at least 500 nm, more preferably still by at least 1 $\mu$m.

The thickness of the lubricant layer increases monotonically over the portion from $p_1$ to $p_2$.

In one embodiment, the thickness of the lubricant layer satisfies the following:

- The portion X is split into n sections of equal length: $M_1$ to $M_n$;
- $M_1$ to $M_n$ are ordered, with $M_1$ starting at $p_1$ and $M_n$ finishing at $p_2$;
- The mean values T of layer thickness over the sections $M_1$ to $M_n$ are $T_1$ to $T_n$ respectively;
- Each mean layer thickness T through the series $T_2$ to $T_n$ is greater than the preceding mean value of T: $T_1$ to $T_{n-1}$ respectively;
- n is an integer of 2 or more. Preferred values for x are 5, 10, 15 and 20.

In one embodiment, the layer extends over at least 70%, more preferably at least 80%, more preferably at least 90%, most preferably at least 94%, most preferably at least 98%, most preferably about 100% of the length $L_B$ of the elongate barrel section. In another embodiment, the lubricant extends over 20 to 60 % of the length $L_B$ elongate barrel section.

In one embodiment, the lubricant layer has a minimum thickness $t_{min}$ determined between $p_1$ and $p_2$ inclusive of at least 60 nm, preferably at least 90 nm, more preferably at least 100 nm, most preferably at least 110 nm.

In one embodiment, the layer has a minimum thickness $t_{min}$ determined between $p_1$ and $p_2$ inclusive which is greater than the mean roughness of the inner surface of the side wall between $p_1$ and $p_2$, preferably at least 20 nm greater, more preferably at least 40 nm, more preferably at least 60 nm greater.

Liquid Pharmaceutical Composition

The receptacle is for pharmaceutical packaging. A preferred receptacle is adapted and arranged to contain a liquid.

A liquid pharmaceutical composition preferably comprises an active compound.

A liquid pharmaceutical composition is a fluid.

A preferred amount of liquid pharmaceutical composition is in the range from 0.1 to 150 ml, preferably in the range from 0.5 to 70 ml, more preferably in the range from 0.8 to 40 ml, most preferably in the range from 1 to 10 ml, most preferably in the range from 2 to 5 ml.

Charge

The receptacle is adapted and arranged to accommodate a charge. A preferred charge is adapted and arranged to be accommodated in the receptacle. The receptacle are preferably complementary such that the charge can be introduced into the interior of the receptacle and such that the charge can move within the interior in a direction parallel to the axis.

A preferred charge is made of an elastic material or comprises a part made of an elastic material. The charge is preferably adapted and arranged to seal a cross-section of the interior. The charge is preferably adapted and arranged to move inside the receptacle, preferably along the axis defined by the elongate extension of the receptacle. When inside the receptacle, movement of the charge is preferably resisted by a frictional force between the charge and an inside surface of the receptacle.

The charge may be attached to an elongate rod adapted and arranged to push or pull the charge in a direction parallel to the axis.

A preferred charge is a plunger.

Charge axial position

When in position in the receptacle, the charge makes contact with the layer or the inner surface or both. The front end of the charge is the point of forwardmost contact, in a direction from $p_A$ to $p_B$, of the charge with the layer or the inner surface. The back end of the charge is the point of backmost contact, in a direction from $p_A$ to $p_B$, of the charge with the layer or the inner surface. The charge axial position is the axial position of the front end of the charge.

The distance between the front end and the back end of the charge is the charge length $L_C$.

Kit

A contribution to at least partially solving one or more of the above-mentioned objects is made by a kit comprising a receptacle according to this disclosure and a charge according to this disclosure.

The charge and the receptacle are preferably complementary such that the charge can be accommodated in the receptacle and can move within the interior in a direction parallel to the axis.

Frictional Forces

The movement of the charge within the receptacle is accompanied by a frictional force between the charge and the inner surface of the side wall and or the layer. The frictional force comprises both a stiction which resists the setting in motion of the charge relative to the receptacle and a dynamic friction which acts whilst the charge is in movement.

The stiction is dependent on the charge axial position. The stiction at a given charge axial position p is preferably determined by starting the charge at charge axial position $p_1 + L_C$, moving the charge from $p_1 + L_C$ at a constant speed of 100 mm/minute, letting the charge come to rest at charge axial position p, for example equidistant between $p_1 + L_C$ and $p_2$, and finally bringing the charge into motion in a direction to towards charge axial position $p_2$. The stiction at charge axial position p is the force required to bring the charge into motion in the final step.

The stiction is preferably lower towards the second end of the receptacle than towards the first end of the receptacle. The stiction at charge axial position $p_2$ is preferably less than the stiction at charge axial position $p_1 + L_C$.

The stiction s preferably reduces monotonically from $p_1 + L_C$ to $p_2$. A method for determining the stiction is presented in the figures.

The stiction is preferably relatively uniform from $p_1 + L_C$ to $p_2$.

Assembly

A contribution to at least partially solving at least one of the above-mentioned objects is made by an assembly comprising a receptacle, a charge and a liquid pharmaceutical composition. The charge is positioned inside the receptacle to seal a cross-section of the interior. The liquid pharmaceutical composition is present in the interior between the cross-section sealed by the charge and an aperture of the receptacle.

In one embodiment, the liquid pharmaceutical composition fills at least 50 vol. % of the interior, preferably at least 70 vol. %, more preferably at least 80 vol. %.

In one embodiment, the liquid pharmaceutical composition fills less than 50 vol. % of the interior, or less than 30 vol. %, or less than 20 vol. %.

The assembly is preferably adapted and arranged such that the liquid pharmaceutical composition can be ejected from the interior by moving the charge in a direction parallel to the axis towards the second end.

A preferred assembly functions as a plunger system in which the liquid pharmaceutical composition can be ejected from the receptacle through movement of the charge.

A preferred receptacle has an attachment means, preferably at an end. A preferred attachment means is adapted and arranged for attaching a needle or tube. A needle or tube may be attached to the receptacle in the assembly.

Luer Fitting

Preferred receptacles, be they receptacles as such or as part of a kit or an assembly, have a Luer fitting. A preferred Luer fitting is compatible with ISO 80369. Preferred Luer fittings are Luer lock fittings and slip tip fittings, preferably Luer lock fittings. In one embodiment, the receptacle has a Luer lock fitting. In another embodiment, the receptacle has a slip tip fitting. A preferred Luer fitting is a male Luer fitting. Preferred Luer lock fittings are one-piece Luer lock fittings and two-piece Luer lock fittings. In one embodiment, the receptacle has a one-piece Luer lock fitting. In another embodiment, the receptacle has a tow-piece Luer lock fitting.

Process for Preparation

A receptacle according to this disclosure may be prepared by providing a receptacle without a layer lubricant and applying a layer of lubricant to the inner surface of the side wall of the receptacle. Preferred methods for applying the layer are spreading and wiping, preferably with a suitable tool.

An assembly may be prepared by the following steps:

- providing a receptacle according to this disclosure;
- introducing a charge into the receptacle, preferably via a first end, preferably via a first aperture;
- introducing a liquid pharmaceutical composition into the interior of the receptacle, preferably via a second end, preferably via a second aperture.

Figures

The invention is now further elucidated by way of figures. The figures are exemplary and do not limit the scope of the invention.

Summary of the Figures

Figure 1 shows a cross-sectional view of a receptacle with a layer of lubricant on an inner surface of a side wall.
Figures 2a to 2m show a multi-push movement of a charge in a receptacle.
Figure 3 shows a cross-sectional view through the receptacle.
Figures 4a to 4e show the process of preparing an assembly and ejecting a pharmaceutical product.
Figure 5 shows a thickness profile of the layer of lubricant.
Figure 6 shows determination of a monotonically increasing thickness profile.
Figure 7 shows a stiction profile for the kit of example 1.

Figure 1 shows a cross-sectional view of a receptacle 100 with a lubricant layer 106 on an inner surface 118 of a side wall 107. The receptacle 100 has a first end 119 and a second end 120. At the first end 119 is a first aperture 102 and an outwardly protruding flange 105. At the second end 120 is a second aperture 103 and an attachment means 104, in this case a screw thread, for attaching a needle fitting. The receptacle 100 has an elongate barrel section 501 extending from an axial position $p_A$ to an axial position $p_B$. In this case, the elongate barrel section 501 has a hollow truncated conical shape with a greater diameter at $p_A$ than at $p_B$. The length of the elongate barrel section 501 is $L_B$. The axis 101 is in the direction of elongate extension of the receptacle 100 and is the axis of rotation of the elongate barrel section 501. The side wall 107 has an inner surface 118, on which is present a layer 106 of lubricant. The layer 106 extends over some, but not all of the side wall 107, not reaching the ends $p_A$ and $p_B$. A portion X of the elongate barrel section 501 runs between axial positions $p_1$ to $p_2$. The portion X is an abstract portion selected according to the criteria presented in the embodiments and claims. The end points $p_1$ and $p_2$ both lie within the bounds of the layer 106. The purpose of selecting the abstract portion X of the elongate barrel section 501 is to ensure that irregularities at the ends of the elongate barrel section 501 are avoided, in particular since the layer 106 may not extend all the way to the ends $p_A$ and $p_B$, as in this example. Axial positions along the receptacle 100 are measured along the axis 101. Axial positions may be given with reference to $p_1$ as a fiduciary zero point. Shown is a general axial position p as well as the internal diameter 118 between of the inner surfaces 118 of the side walls 107 at that axial position. The thicknesses $t_1$ at axial position $p_1$ and $t_2$ at axial position $p_2$ are shown. According to this disclosure, $t_2$ is greater than $t_1$. In the case shown, $t_1$ and $t_2$ are rather similar, but the difference could be much more marked.

Figures 2a to 2m show a multi-push movement of a charge 203 in a receptacle 100. The series of figures demonstrates how the charge 203 may be pushed along the axis 101 of the receptacle 100 in multiple pushes in between which the charge 203 comes to rest at least once on its path along the axis 101. In the process shown, the charge 203 comes to rest at two intermediate axial positions along its path. The process presented can also be used to construct a profile of dynamic friction and stiction for the receptacle/charge combination as a function of distance along the axis 101.

Figure 2a shows a receptacle 100 ready for multi-push movement of a charge 203. The receptacle 100 is in this case a syringe barrel, having a major hollow truncated conical section (The angle of inclination of the truncated cone is very slight and the elongate barrel section has been represented as a cylinder for simplicity of view). An axis 101 is in the direction of the elongate extension of the receptacle 100. The axis 101 is the axis of rotation of the conical/cylindrical section. At a first end 119 of the receptacle 100 is a first aperture 102 and an outwardly protruding flange 105. At a second end 120 of the receptacle is a second aperture 103 which exhibits a narrowing and is adapted and arranged to attach a needle. The first aperture 102 has a greater diameter area than the second aperture 103. The receptacle 100 has a cylindrical side wall 107 and an interior 121. A layer 106 of lubricant is present on the inner surface 118 of the side wall 107. The lubricant layer 106 is present along the majority of the side wall 107. In this case, the portion X covers most of the lubricant layer 107, with $p_1$ and $p_2$ slightly removed from the ends of the layer 107 in order to avoid end effects. The layer 106 of lubricant has a thickness 210, shown at a general axial position along the axis 101. For ease of depiction, the layer 106 is shown roughly uniform, but the preferred thickness profile is described elsewhere in this document. Present in the interior 121, towards the first end 119 of the receptacle 100, is a charge 203. In this case, the charge 203 is a stopper of elastomer material having an attached elongate rod 202. The charge 203 has ribbed side walls. The elongate rod 202 can be used to push the charge 203 along the axis 101 in the manner of a plunger. The axial position of the charge 203 is defined as the axial position of the front end of the charge 203. The charge 203 is shown in an initial axial position 201 in which the back end of the charge 203 is at $p_1$. In the initial state presented, no force is applied to the charge via the elongate rod 202 and the charge 203 is at rest.

Figure 2b shows the receptacle 100 of figure 1, in which a pushing force 207 is applied to the elongate rod 202 in a direction along the axis 101. The force 207 is transferred to the charge 203. In the figure, the force 207 is inferior to the stiction at the axial position 201 and the charge 203 is at rest, with the pushing force 207 cancelled out by the static frictional force between the charge 203 and the side walls 207 / lubricant layer 106. The stiction at the axial position 201 is determined as the force 207 at which the charge 203 starts to move along the axis 101.

Figure 2c shows the receptacle 100 immediately after the pushing force 207 in figure 2b exceeds the stiction at axial position 201 to put the charge 203 into motion. The charge 203 is depicted at the axial position 201, but in motion 208 along the axis 101. The pushing force 207 is equal to the dynamic friction at axial position 201 and the charge 203 is in a state of constant velocity along the axis 101. The charge 203 is moved between rest axial positions at a constant speed of 100 mm/min.

Figure 2d shows the receptacle 100 subsequent to that of figure 2c, in which the charge 203 has travelled a distance from 201 to an intermediate axial position 204 along the axis 101. The charge 203 is still in motion 208 with constant velocity of 100 mm/min with the pushing force 207.

Figure 2e shows the receptacle 100 in which the pushing force 207 has been released at axial position 204. The charge is at rest at the axial position 204.

Figures 2e to 2i are analogous to figures 2a to 2e. The charge 203 is initially at rest at axial position 204 (figure 2e) and is set in motion 208 by a force 207 exceeding the stiction at axial position 204 (figure 2f). The charge 203 starts moving 208 along the axis 101 at axial position 204 (figure 2g) and arrives in motion 208 at a further intermediate axial position 205 (figure 2h), where the pushing force 207 is released and the charge 203 comes to a stop (figure 2i).

The speed between rest axial positions is maintained constant at 100 mm/min.

Figures 2i to 2m are analogous to figures 2a to 2e and figures 2e to 2i respectively. The charge 203 is initially at rest at axial position 205 (figure 2i) and is set in motion 208 by a force 207 exceeding the stiction at axial position 205 (figure 2j). The charge 203 starts moving 208 along the axis 101 at axial position 205 (figure 2k) and arrives in motion 208 at the final axial position 206 (figure 2l), where the pushing force 207 is released and the charge 203 comes to a stop (figure 2m). The axial position 206, is where the front end of the charge has arrived at axial position $p_2$, close to the end of the lubricated layer 106 and is in its final rest axial position. The speed between rest axial positions is maintained constant at 100 mm/min.

The stiction at points 201, 204 and 205 are measured at the following points:

| 201 | Figure 2b |
| --- | --- |
| 204 | Figure 2f |
| 205 | Figure 2j |

Figure 3 shows a cross-sectional view through the receptacle 100 at an axial position p along the axis 101. The side wall 107 and the lubricant layer 106 are shown as concentric circular bands. The thickness 301 of the side wall 107 and the thickness 302 of the lubricant layer 106 are each shown at 8 equidistant points around the circle. A thickness of a side wall 107 or a layer 106 of lubricant at an axial position p is a mean of the thickness around the circle. This is measured as the mean of a number of equally spaced sample points around the circle, in this case 8.

Figures 4a to 4e show the process of preparing an assembly and ejecting a pharmaceutical product 401.

Figure 4a shows a receptacle 100, ready for forming an assembly. The receptacle 100 has an axis of rotation 101, a side wall 107 and an interior 121. A layer 106 of lubricant is present on the inside of the receptacle 100. The receptacle 100 has a first aperture 102 and a second aperture 103.

Figure 4b shows the receptacle 100 of figure 4a with a charge 203 located in the interior 121. The front end of the charge 203 is at an axial position 201 along the axis, close to the first aperture 102. The back end of the charge 203 is at $p_1$ close to the start of the lubricant layer 107.

Figure 4c shows the receptacle 100 of figure 4b after having been filled with a liquid pharmaceutical composition 401. The liquid pharmaceutical composition 401 is located in the interior 121 between the front end of the charge 201 and the second aperture 103. In this form, the receptacle 100, the charge 203 and the liquid pharmaceutical composition 401 constitute and assembly.

Figure 4d shows the assembly of figure 4c in which a pushing force 207 is applied to push the charge 203 along the axis 101 in a direction from the first aperture 102 towards the second aperture 103. The motion 208 of the charge 203 forces the liquid pharmaceutical composition 401 to be ejected 402 from the receptacle 100.

Figure 4e shows the assembly of figure 4d once the charge 203 has travelled along the axis 101 in a direction from the first aperture 102 towards the second aperture 103 to arrive at axial position $p_2$ near the end of the layer 106 of lubricant. The liquid pharmaceutical composition 401 has been ejected via the second aperture 103 and only a small quantity remains in the tip of the receptacle at the second aperture 103.

Figure 5 shows a thickness profile 200 of the layer 106 of lubricant. The thickness t increases from a value of $t_1$ 122 at an axial position $p_1$ 115 to a value of $t_2$ 123 at an axial position $p_2$ 117. The gradient of the thickness against axial position is not negative anywhere along the extent from $p_1$ 115 to $p_2$ 117. The profile is monotonic.

Figure 6 shows determination of a monotonically increasing thickness profile. The stretch from $p_1$ to $p_2$ has been split into 10 sections $M_1$ to $M_{10}$ of equal size. The mean thickness over the sections $M_1$ to $M_{10}$ is $T_1$ to $T_{10}$ respectively. Despite sharp variations of the thickness profile on a short range due to roughness, the longer-range monotonicity of the thickness profile is evident from the strictly ascending ordering from $T_1$ to $T_{10}$, without descending, as can be seen on the t axis.

Figure 7 shows a stiction profile for the kit of example 1. The stiction profile is determined using the procedure as presented in the figures 2a to 2m, except with 3 intermediate rest points at 15, 30 and 45 mm from the start point 201. The test was performed 10 times with fresh kits (all 10 lines shown in the figure). In each run, a smooth stiction profile was observed, mildly decreasing from around 4.8-6.2 N at 201 to around 2.8-3.8 N at 45 mm from $c_\alpha$. The gently and monotonically decreasing stiction profile is well suited to be employed in a multi-dose syringe. As can be seen, the transition between stiction and dynamic friction is smooth, allowing better control of multi-dose ejection.

<u>Test Methods</u>

<u>Layer thickness</u>

The thickness of the layer is determined by optical interference measurements using the RapID Explorer available from rap.ID Particle Systems GmbH. Measurements are taken from outside the receptacle through the side wall. The device is operated with the proprietary software and in accordance with the 2014 proprietary instruction manual.

<u>Surface Roughness</u>

Surface roughness of the inner surface of the side wall is measured using a white-light interference microscope. An area of the sample of 2$\mu$m by 2$\mu$m is scanned in tapping mode, scanning the area with 256 lines per picture and 256 dots per line. The scan rate is 0.7 Hz. The cantilever has a tip with a tip radius of $\leq$ 10nm. The sample's topography is measured by evaluating the change of the amplitude of the oscillating cantilever when scanning the surface. The raw data is levelled by a line fit, using a 3$^{rd}$ order polynomial fit. The root mean squared roughness $R_{rms}$ is calculated by the

$$R_{rms} = \sqrt{\frac{1}{n}\sum_{i=1}^{n} y_i^2}$$

AFM's software using the formula , where n = 256 * 256 = 65536 and $y_i$ is the height value at each of the 65536 measured positions.

<u>Examples</u>

The following examples are for further elucidation of the invention and do not limit the scope of the claimed invention.

A receptacle was provided according to figure 1. The receptacles was a 1 ml lg TopPac available from Schott AG Germany. The inner surface of the side walls of the elongate barrel section was coated with a silicone coating, extending up to 1 mm from each of the two ends of the elongate barrel section. The thickness profile of the applied layer was according to table 1. The receptacle was tested by introducing a charge made of elastomer with an attached elongate rod according to figure 2a, charge FM 257/2 available from Dedecke GmbH, Germany. The process similar to that displayed in figures 2a to 2m, starting at 5 mm along the barrel and with 3 intermediate stop points, at 15, 30 and 45 mm along the barrel, was performed to determine the stiction along the barrel.

The root mean square of the roughness of the inner surface of the side wall over the section from $p_1$ to $p_2$ was determined to be 62 nm.

Table 1

| Example | Distance along barrel [mm] | | | | | | |
|---|---|---|---|---|---|---|---|
| | 0 | 10 | 20 | 30 | 40 | 50 | 60 |
| | Thickness t [nm] | | | | | | |
| 1 | 71 | 279 | 722 | 1014 | 1304 | 1708 | 1863 |
| 2 | 74 | 511 | 1423 | 1982 | 2356 | 2870 | 3987 |
| 3* | 2102 | 1677 | 1298 | 1012 | 697 | 311 | 69 |
| 4* | 956 | 944 | 963 | 961 | 948 | 951 | 960 |

Table 2

| Example | Distance along barrel [mm] | | | |
|---|---|---|---|---|
| | 5 | 15 | 30 | 45 |
| | Stiction [N] | | | |
| 1 | 6.2 | 5.0 | 4.3 | 3.7 |
| 2 | 5.7 | 4.6 | 3.8 | 2.8 |

(continued)

| Example | Distance along barrel [mm] | | | |
|---|---|---|---|---|
| | 5 | 15 | 30 | 45 |
| | Stiction [N] | | | |
| 3* | 5.8 | 6.4 | 7.1 | 7.8 |
| 4* | 6.0 | 6.1 | 6.1 | 6.2 |
| * Not according to the present invention. | | | | |

Reference List

| | |
|---|---|
| 100 | Receptacle |
| 101 | Axis |
| 102 | First aperture |
| 103 | Second aperture |
| 104 | Attachment means at front end of receptacle |
| 105 | Outwardly protruding flange of receptacle |
| 106 | Layer of lubricant |
| 107 | Side wall of receptacle |
| 118 | Inner surface of side wall |
| 119 | First end of receptacle |
| 120 | Second end of receptacle |
| 121 | Interior of receptacle |
| 201 | Initial charge axial position |
| 202 | Elongate rod for pushing charge |
| 203 | Charge |
| 204 | First intermediate charge axial position |
| 205 | Second intermediate charge axial position |
| 206 | Final charge axial position |
| 207 | Pushing force |
| 208 | Movement of charge along axis |
| 210 | Thickness of layer at a general axial position |
| 301 | Thicknesses of the side wall |
| 302 | Thicknesses of the layer |
| 401 | Liquid pharmaceutical composition |
| 402 | Ejection of liquid pharmaceutical composition |
| 501 | Elongate barrel section |

**Claims**

1. A receptacle (100) for pharmaceutical packaging, the receptacle (100) having an elongate barrel section (501) and comprising a first aperture (102) at a first end (119) and a second aperture (103) at a second end (120), wherein:

   a. the elongate barrel section (501) has a direction of elongate extension and an axis (101) in the direction of elongate extension;
   b. an axial position p is determined along the axis (101);
   c. the elongate barrel section (501) extends from an axial position $p_A$ to an axial position $p_B$, wherein the receptacle (100) is adapted and arranged for ejecting a liquid pharmaceutical composition from the second end (120) and wherein $p_B$ is closer to the second end than is $p_A$;
   d. an elongate barrel section length $L_B$ is the distance between $p_A$ and $p_B$;
   e. the receptacle (100) has a side wall (107) extending over the elongate barrel section (501), the side wall (107) having an inner surface (118) bordering an interior (121), the interior (121) having a diameter (113);

f. a layer of a lubricant (106) is located on at least a part of the inner surface (118);
g. at a given axial position p on the axis (101) between $p_A$ and $p_B$, the following:

    i. the thickness of the side wall (107),
    ii. the thickness of the layer (106), and
    iii. the diameter of the interior (113),

are each determined as an angular mean in a cross-sectional plane perpendicular to the axis (101) at the axial position p;

h. a portion X of the axis (101) extends from an axial position $p_1$ to an axial position $p_2$ such that the following criteria are satisfied:

    i. both $p_1$ and $p_2$ lie between $p_A$ and $p_B$,
    ii. a portion length Lx is the distance between $p_1$ and $p_2$,
    iii. Lx is at least a quarter of $L_B$,
    iv. the layer (106) extends over the entire portion X, and
    v. a thickness $t_1$ of the layer (106) at $p_1$ is less than a thickness $t_2$ of the layer (106) at $p_2$;

wherein $p_1$ is closer to $p_A$ than is $p_2$ and wherein the thickness of the layer (106) increases monotonically over the portion from $p_1$ to $p_2$;

i. criteria i., ii., iii. and iv.:

    i. The length $L_B$ is in the range from 3 cm to 20 cm;
    ii. The mean value of the diameter (113) of the interior (121) determined over the range $p_A$ to $p_B$ is in the range from 0.4 to 4 cm;
    iii. The mean thickness of the sidewall (107) determined over the range $p_A$ to $p_B$ is in the range from 0.3 mm to 4 mm; and
    iv. The volume of the interior (121) is in the range from 0.1 to 150 ml;

are satisfied.

2. The receptacle (100) according to claim 1, wherein the ratio of the thicknesses $t_1:t_2$ is in the range from 1:1.1 to 1:100.

3. The receptacle (100) according to claim 1 or 2, wherein the lubricant comprises one or more silicone oils.

4. The receptacle (100) according to claim 3, wherein the one or more silicone oils are at least partially contained in a matrix.

5. The receptacle (100) according to any of the preceding claims, wherein the interior (121) is cylindrical or truncated conical over the elongate barrel section (501).

6. The receptacle (100) according to any of the preceding claims, wherein the internal diameter (113) at $p_1$ is $d_1$ and the internal diameter (113) at $p_2$ is $d_2$ and $d_1$ is greater than $d_2$.

7. The receptacle (100) according to any of the preceding claims, comprising an aperture and an attachment means (104) at the aperture.

8. The receptacle (100) according to any of the preceding claims, wherein the layer (106) has a minimum thickness $t_{min}$ determined in the portion X of at least 60 nm.

9. A kit comprising as kit parts:

    a. the receptacle (100) according to any of the preceding claims, and
    b. a charge (203);

wherein the charge (203) is adapted and arranged to be positioned in the interior (121) such that:

    i. the charge seals a cross section of the interior (121) between the inner surfaces (118) of the side walls (107);

ii. the charge (203) has a front end at the axial position closest to $p_B$ at which the charge contacts the layer (106) or the inner surface (118);

iii. the charge (203) has a back end at the axial position closest to $p_A$ at which the charge contacts the layer (106) or the inner surface (118);

iv. a length of the charge Lc is the distance between the front end and the back end;

v. the charge (203) has a charge axial position, being the axial position of the front end;

vi. the charge (203) is movable in a direction parallel to the axis (101) with a stiction s, s being a function of the charge axial position.

**10.** The kit according to claim 9, wherein, for charge axial positions in the range from $p_1$ + Lc to $p_2$, the stiction s has a maximum value $s_{max}$, a minimum value $s_{min}$ and the value of Smin / Smax is 70 % or more.

**11.** The kit according to claim 9 or 10, wherein the stiction at charge axial position $p_2$ is equal to or less than the stiction at charge axial position $p_1$ + Lc.

**12.** An assembly comprising a receptacle (100) according to any of the claims 1 to 8 and a charge (203), wherein:

a. the charge (203) is positioned in the interior (121) sealing a cross section of the interior (121);

b. the interior (121) contains a liquid pharmaceutical composition, located between the sealed cross section and the aperture (103); and

c. the assembly is adapted and arranged for the liquid pharmaceutical composition to be ejected through the aperture (103) by movement of the charge (203) in a direction parallel to the axis (101).

**13.** A process for preparing a receptacle (100) according to any of the claims 1 to 8, a kit according to any of the claims 9 to 11, or an assembly according to claim 12, comprising a process step of applying the layer (106) of lubricant by spreading with a spreading tool.

**Patentansprüche**

**1.** Behälter (100) für pharmazeutische Verpackungen, wobei der Behälter (100) einen länglichen Trommelabschnitt (501) aufweist und eine erste Öffnung (102) an einem ersten Ende (119) und eine zweite Öffnung (103) an einem zweiten Ende (120 ) umfasst, wobei:

a. der längliche Zylinderabschnitt (501) eine Richtung der Längserstreckung und eine Achse (101) in der Richtung der Längserstreckung aufweist;

b. wird eine axiale Position p entlang der Achse (101) bestimmt;

c. der längliche Zylinderabschnitt (501) sich von einer axialen Position $p_A$ zu einer axialen Position $p_B$ erstreckt, wobei das Behältnis (100) angepasst und angeordnet ist, um eine flüssige pharmazeutische Zusammensetzung aus dem zweiten Ende (120) auszustoßen, und wobei $p_B$ näher an dem zweiten Ende ist als $p_A$ ;

d. die Länge $L_B$ ist der Abstand zwischen $p_A$ und $p_B$ ;

e. das Gefäß (100) eine Seitenwand (107) aufweist, die sich über den länglichen Zylinderabschnitt (501) erstreckt, wobei die Seitenwand (107) eine Innenfläche (118) aufweist, die einen Innenraum (121) begrenzt, wobei der Innenraum (121) einen Durchmesser (113) aufweist;

f. eine Schicht aus einem Schmiermittel (106) auf mindestens einem Teil der Innenfläche (118) angeordnet ist;

g. bei einer gegebenen axialen Position p auf der Achse (101) zwischen $p_A$ und p , folgendes: $_B$

i. die Dicke der Seitenwand (107),

ii. die Dicke der Schicht (106), und

iii. der Durchmesser des Innenraums (113),

jeweils als Winkelmittel in einer Querschnittsebene senkrecht zur Achse (101) an der axialen Position p bestimmt werden;

h. ein Abschnitt X der Achse (101) sich von einer axialen Position $p_1$ bis zu einer axialen Position $p_2$ erstreckt, so dass die folgenden Kriterien erfüllt sind:

i. sowohl $p_1$ als auch $p_2$ liegen zwischen $p_A$ und $p_B$ ,

ii. eine Teillänge $L_X$ ist der Abstand zwischen $p_1$ und $p_2$ ,

iii. $L_X$ ist mindestens ein Viertel von $L_B$,

iv. die Schicht (106) erstreckt sich über den gesamten Abschnitt X, und

v. eine Dicke $t_1$ der Schicht (106) bei $p_1$ geringer ist als eine Dicke $t_2$ der Schicht (106) bei $p_2$ ;

wobei $p_1$ näher an $p_A$ liegt als $p_2$ und , wobei die Dicke der Schicht (106) über den Abschnitt von $p_1$ bis $p_2$ monoton zunimmt;

i. die Kriterien i., ii., iii. und iv:

i. Die Länge $L_B$ liegt im Bereich von 3 cm bis 20 cm;

ii. Der Mittelwert des Durchmessers (113) des Innenraums (121), der über den Bereich $p_A$ bis $p_B$ ermittelt wird, liegt im Bereich von 0,4 bis 4 cm;

iii. die über den Bereich $p_A$ bis $p_B$ ermittelte mittlere Dicke der Seitenwand (107) im Bereich von 0,3 mm bis 4 mm liegt; und

iv. Das Volumen des Innenraums (121) liegt im Bereich von 0,1 bis 150 ml; erfüllt sind.

2. Das Gefäß (100) nach Anspruch 1, wobei das Verhältnis der Dicken $t_1 : t_2$ im Bereich von 1:1,1 bis 1:100 liegt.

3. Das Gefäß (100) nach Anspruch 1 oder 2, wobei das Schmiermittel ein oder mehrere Silikonöle umfasst.

4. Gefäß (100) nach Anspruch 3, wobei das eine oder die mehreren Silikonöle zumindest teilweise in einer Matrix enthalten sind.

5. Gefäß (100) nach einem der vorhergehenden Ansprüche, wobei der Innenraum (121) über dem langgestreckten Rohrabschnitt (501) zylindrisch oder kegelstumpfförmig ist.

6. Das Gefäß (100) nach einem der vorhergehenden Ansprüche, wobei der Innendurchmesser (113) bei $p_1$ $d_1$ und der Innendurchmesser (113) bei $p_2$ $d_2$ ist und $d_1$ größer als $d_2$ ist.

7. Behältnis (100) nach einem der vorhergehenden Ansprüche, mit einer Öffnung und einem Befestigungsmittel (104) an der Öffnung.

8. Gefäß (100) nach einem der vorhergehenden Ansprüche, wobei die Schicht (106) eine im Bereich X ermittelte Mindestdicke $t_{min}$ von mindestens 60 nm aufweist.

9. Ein Bausatz, der als Bausatzteile enthält:

a. das Gefäß (100) nach einem der vorhergehenden Ansprüche, und

b. eine Ladung (203);

wobei die Ladung (203) so angepasst und angeordnet ist, dass sie im Innenraum (121) positioniert wird, so dass:

i. die Ladung dichtet einen Querschnitt des Innenraums (121) zwischen den Innenflächen (118) der Seitenwände (107) ab;

ii. die Ladung (203) hat ein vorderes Ende an der axialen Position, die $p_B$ am nächsten liegt, an der die Ladung die Schicht (106) oder die Innenfläche (118) berührt;

iii. die Ladung (203) hat ein hinteres Ende an der axialen Position, die $p_A$ am nächsten liegt, an der die Ladung die Schicht (106) oder die Innenfläche (118) berührt;

iv. eine Länge der Ladung $L_C$ ist der Abstand zwischen dem vorderen und dem hinteren Ende;

v. die Ladung (203) hat eine axiale Position der Ladung, die die axiale Position des vorderen Endes ist;

vi. die Ladung (203) ist in einer Richtung parallel zur Achse (101) mit einer Reibung s beweglich, wobei s eine Funktion der axialen Position der Ladung ist.

10. Bausatz nach Anspruch 9, **dadurch gekennzeichnet, dass** für axiale Ladungspositionen im Bereich von $p_1 + L_C$ bis $p_2$ die Reibung s einen Maximalwert $s_{max}$ , einen Minimalwert $s_{min}$ hat und der Wert von $s_{min} / s_{max}$ 70 % oder mehr beträgt.

11. Bausatz nach Anspruch 9 oder 10, wobei die Reibung an der axialen Position der Ladung $p_2$ gleich oder geringer ist als die Reibung an der axialen Position der Ladung $p_1 + L_C$ .

**EP 3 824 926 B1**

12. Baugruppe mit einem Gefäß (100) nach einem der Ansprüche 1 bis 8 und einer Ladung (203), wobei:

a. die Ladung (203) im Innenraum (121) angeordnet ist und einen Querschnitt des Innenraums (121) abdichtet;
b. der Innenraum (121) eine flüssige pharmazeutische Zusammensetzung enthält, die sich zwischen dem abgedichteten Querschnitt und der Öffnung (103) befindet; und
c. die Anordnung so angepasst und angeordnet ist, dass die flüssige pharmazeutische Zusammensetzung durch eine Bewegung der Ladung (203) in einer Richtung parallel zur Achse (101) durch die Öffnung (103) ausgestoßen wird.

13. Verfahren zur Herstellung eines Gefäßes (100) nach einem der Ansprüche 1 bis 8, eines Kits nach einem der Ansprüche 9 bis 11 oder einer Baugruppe nach Anspruch 12, umfassend einen Verfahrensschritt des Auftragens der Schmiermittelschicht (106) durch Verteilen mit einem Verteilerwerkzeug.

**Revendications**

1. Un récipient (100) pour emballage pharmaceutique, le récipient (100) ayant une section allongée (501) et comprenant une première ouverture (102) à une première extrémité (119) et une seconde ouverture (103) à une seconde extrémité (120) , dans lequel :

a. la section allongée du canon (501) a une direction d'extension allongée et un axe (101) dans la direction d'extension allongée ;
b. une position axiale p est déterminée le long de l'axe (101) ;
c. la section allongée du cylindre (501) s'étend d'une position axiale $p_A$ à une position axiale $p_{,B}$ dans laquelle le récipient (100) est adapté et agencé pour éjecter une composition pharmaceutique liquide de la deuxième extrémité (120) et dans laquelle $p_B$ est plus proche de la deuxième extrémité que ne l'est $p_A$ ;
d. une longueur de section de canon allongée $L_B$ est la distance entre $p_A$ et $p_B$ ;
e. le récipient (100) possède une paroi latérale (107) s'étendant sur la section allongée du canon (501), la paroi latérale (107) ayant une surface intérieure (118) bordant un intérieur (121), l'intérieur (121) ayant un diamètre (113) ;
f. une couche de lubrifiant (106) est située sur au moins une partie de la surface intérieure (118) ;
g. à une position axiale donnée p sur l'axe (101) entre $p_A$ et $p_B$, les éléments suivants

i. l'épaisseur de la paroi latérale (107),
ii. l'épaisseur de la couche (106), et
iii. le diamètre de l'intérieur (113),

sont chacun déterminés comme une moyenne angulaire dans un plan de coupe perpendiculaire à l'axe (101) à la position axiale p ;
h. une partie X de l'axe (101) s'étend d'une position axiale $p_1$ à une position axiale $p_2$ de telle sorte que les critères suivants soient satisfaits :

i. les $p_1$ et $p_2$ sont tous deux situés entre les $p_A$ et $p_B$ ,
ii. une portion de longueur $L_X$ est la distance entre $p_1$ et $p_2$ ,
iii. $L_X$ représente au moins un quart de $L_B$ ,
iv. la couche (106) s'étend sur la totalité de la portion X, et
v. une épaisseur $t_1$ de la couche (106) à $p_1$ est inférieure à une épaisseur $t_2$ de la couche (106) à $p_2$ ;

dans lequel $p_1$ est plus proche de $p_A$ que de $p_2$ et dans lequel l'épaisseur de la couche (106) augmente de façon monotone sur la portion allant de $p_1$ à $p_2$ ;
i. critères i., ii., iii. et iv :

i. La longueur $L_B$ est comprise entre 3 cm et 20 cm ;
ii. la valeur moyenne du diamètre (113) de l'intérieur (121) déterminée dans l'intervalle $p_A$ à $p_B$ est comprise entre 0,4 et 4 cm ;
iii. l'épaisseur moyenne de la paroi latérale (107) déterminée sur la plage $p_A$ à $p_B$ est comprise entre 0,3 mm et 4 mm ; et
iv. Le volume de l'intérieur (121) est compris entre 0,1 et 150 ml ;

sont satisfaits.

2. Le récipient (100) selon la revendication 1, dans lequel le rapport des épaisseurs $t_1 : t_2$ est compris entre 1:1,1 et 1:100.

3. Le récipient (100) selon la revendication 1 ou 2, dans lequel le lubrifiant comprend une ou plusieurs huiles de silicone.

4. Le récipient (100) selon la revendication 3, dans lequel une ou plusieurs huiles de silicone sont au moins partiellement contenues dans une matrice.

5. Le récipient (100) selon l'une quelconque des revendications précédentes, dans lequel l'intérieur (121) est cylindrique ou tronconique sur la section allongée du baril (501).

6. Le récipient (100) selon l'une quelconque des revendications précédentes, dans lequel le diamètre interne (113) à $p_1$ est $d_1$ et le diamètre interne (113) à $p_2$ est $d_2$ et $d_1$ est supérieur à $d_2$.

7. Le récipient (100) selon l'une des revendications précédentes, comprenant une ouverture et un moyen de fixation (104) à l'ouverture.

8. Le récipient (100) selon l'une quelconque des revendications précédentes, dans lequel la couche (106) a une épaisseur minimale $t_{min}$ déterminée dans la portion X d'au moins 60 nm.

9. Un kit comprenant comme pièces de kit :

   a. le récipient (100) selon l'une quelconque des revendications précédentes, et
   b. une charge (203) ;

   dans lequel la charge (203) est adaptée et agencée pour être positionnée dans l'intérieur (121) de telle sorte que :

   i. la charge scelle une section transversale de l'intérieur (121) entre les surfaces intérieures (118) des parois latérales (107) ;
   ii. la charge (203) a une extrémité avant à la position axiale la plus proche de $p_B$ où la charge entre en contact avec la couche (106) ou la surface intérieure (118) ;
   iii. la charge (203) a une extrémité arrière à la position axiale la plus proche de $p_A$ où la charge entre en contact avec la couche (106) ou la surface intérieure (118) ;
   iv. une longueur de la charge $L_C$ est la distance entre l'extrémité avant et l'extrémité arrière ;
   v. la charge (203) a une position axiale de charge, qui est la position axiale de l'extrémité avant ;
   vi. la charge (203) est mobile dans une direction parallèle à l'axe (101) avec une friction s, s étant une fonction de la position axiale de la charge.

10. Le kit selon la revendication 9, dans lequel, pour les positions axiales de charge dans la plage de $p_1 + L_C$ à $p_2$, la friction s a une valeur maximale $s_{max}$, une valeur minimale $s_{min}$ et la valeur de $s_{min} / s_{max}$ est de 70 % ou plus.

11. Le kit selon la revendication 9 ou 10, dans lequel la rigidité à la position axiale de charge $p_2$ est égale ou inférieure à la rigidité à la position axiale de charge $p_1 + L_C$.

12. Un ensemble comprenant un réceptacle (100) selon l'une quelconque des revendications 1 à 8 et une charge (203), dans lequel :

    a. la charge (203) est positionnée dans l'intérieur (121) et scelle une section transversale de l'intérieur (121) ;
    b. l'intérieur (121) contient une composition pharmaceutique liquide, située entre la section transversale scellée et l'ouverture (103) ; et
    c. l'ensemble est adapté et disposé pour que la composition pharmaceutique liquide soit éjectée à travers l'ouverture (103) par le mouvement de la charge (203) dans une direction parallèle à l'axe (101).

13. Un procédé de préparation d'un récipient (100) selon l'une quelconque des revendications 1 à 8, d'un kit selon l'une quelconque des revendications 9 à 11, ou d'un assemblage selon la revendication 12, comprenant une étape d'application de la couche (106) de lubrifiant par étalement à l'aide d'un outil d'étalement.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4767414 A
- EP 0920879 B1
- WO 2015181173 A1
- EP 2216061 A1